(19) Europäisches Patentamt
European Patent Office
Office européen des brevets

(11) Numéro de publication: **0 098 005**
**A2**

(12) # DEMANDE DE BREVET EUROPEEN

(21) Numéro de dépôt: 83200905.4

(22) Date de dépôt: 20.06.83

(51) Int. Cl.³: **C 07 D 273/04**

(30) Priorité: 29.06.82 LU 84242

(43) Date de publication de la demande: 11.01.84 Bulletin 84/2

(84) Etats contractants désignés: BE CH DE FR GB IT LI NL

(71) Demandeur: SOCIETE CARBOCHIMIQUE en abrégé "CARBOCHIM"
avenue de la Renaissance 12
B-1040 Bruxelles(BE)

(72) Inventeur: Lambert, Pierre
Rue de la Citronnelle, 19A
B-1348 Louvain-la-Neuve(BE)

(72) Inventeur: De Aguirre-Otegui, Ignacio
Chemin des Maréchaux, 7
B-1350 Wavre-Limal(BE)

(74) Mandataire: De Brabanter, Maurice et al,
Bureau VANDER HAEGHEN 63 Avenue de la Toison d'Or
B-1060 Bruxelles(BE)

(54) **Sels d'oxadiazines 1,3,5 triones 2,4,6, oxadiazines 1,3,5 triones 2,4,6 et leur préparation.**

(57) L'invention concerne de nouveaux sels d'oxadiazines 1,3,5 triones 2,4,6 et leur préparation par réaction d'un isocyanate ou d'un polyisocyanate organique avec un cyanate salin tel qu'un cyanate de métal ou d'ammonium, phosphonium ou arsonium, et avec de l'anhydride carbonique.

L'invention concerne aussi de nouvelles oxadiazines 1,3,5 triones 2,4,6 moléculaires asymétrques et leur préparation par réaction d'un sel d'oxadiazine 1,3,5 trione 2,4,6 avec un halogénure ou un sulfate organique ou encore un acide.

EP 0 098 005 A2

# SELS D'OXADIAZINES 1,3,5 TRIONES 2,4,6, OXADIAZINES 1,3,5 TRIONES 2,4,6 ET LEUR PREPARATION

La présente invention est relative à de nouveaux sels d'oxadiazines 1,3,5 triones 2,4,6, à de nouvelles oxadiazines 1,3,5 triones 2,4,6 et à leur préparation.

Les nouveaux sels d'oxadiazines 1,3,5 triones 2,4,6 faisant l'objet de la présente invention, appelés aussi oxadiazines 1,3,5 triones 2,4,6, sont représentés par la formule générale (I) ci-dessous :

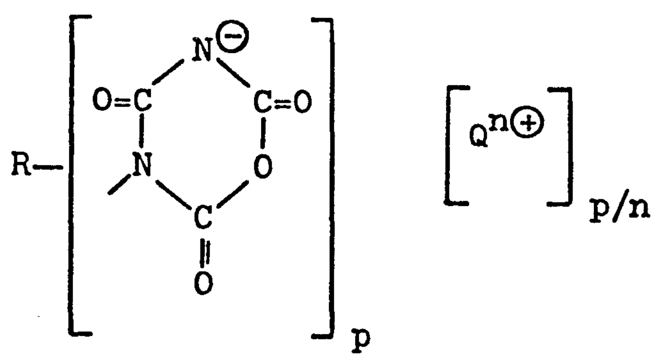

(I)

dans laquelle

R désigne un radical hydrocarboné, substitué ou non, mono- ou polyvalent ;

Q désigne un métal ou un groupement contenant un nombre n de fonctions ammonium, phosphonium ou arsonium, non substituées ou mono-, di-, tri- ou tétrasubstituées ;

p est un nombre entier au moins égal à 1 ;

n désigne le nombre d'oxydation du métal ou le nombre de fonctions ammonium, phosphonium ou arsonium présentes dans le groupement Q.

Les nouvelles oxadiazines 1,3,5 triones 2,4,6 moléculaires asymétriques de la présente invention sont représentées par la formule générale (II) ci-dessous :

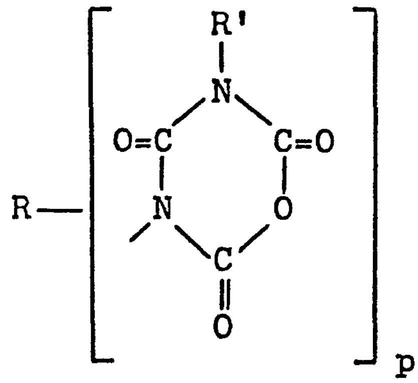

(II)

dans laquelle R et p ont la même signification que dans les composés de formule générale (I) et R', qui est différent de R, désigne un radical hydrocarboné éventuellement substitué, l'hydrogène, un halogène, un radical chlorocarbonyle, carbamoyle, sulfamoyle ou sulfonyle.

Les sels d'oxadiazines 1,3,5 triones 2,4,6 de formule (I) suivant la présente invention sont des produits nouveaux, dont la préparation n'a pas encore été décrite dans la littérature.

En ce qui concerne les oxadiazines 1,3,5 triones 2,4,6 moléculaires, il est connu, par les travaux de K.H. Slotta et R. Tschesche, Ber., 60, 295 (1927) et A. Etienne et B. Bonte, Bull. Soc. Chim. France 7-8, 1497 (1974), de préparer des oxadiazines 1,3,5 triones 2,4,6 moléculaires disubstituées symétriques de formule générale :

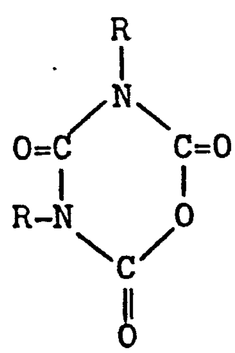

dans lesquelles les substituants portés par les atomes d'azote en position 3 et 5 sont identiques, à partir de deux molécules d'isocyanate aliphatique, de formule RNCO, et d'anhydride carbonique, en présence de trialcoylphosphine comme catalyseur. Par ailleurs, les brevets français n° 1.509.843 (1966) et allemand n° 1.670.666 (1975) décrivent la préparation, suivant le même principe, d'oxadiazines 1,3,5 triones moléculaires portant des groupements isocyanates libres, par action de l'anhydride carbonique sur des diisocyanates aliphatiques.

Ce type de procédé présente notamment, comme inconvénients, l'emploi de dérivés toxiques du phosphore comme catalyseurs et la nécessité d'utiliser, pour la préparation d'une mole d'oxadiazine, deux moles d'isocyanate aliphatique qui constitue un réactif coûteux.

Les oxadiazines 1,3,5 triones 2,4,6 asymétriques de formule (II), c'est-à-dire portant des substituants différents sur les positions 3 et 5, sont nouvelles; toute tentative de préparation de ces oxadiazines 1,3,5 triones 2,4,6 asymétriques par réaction d'isocyanates et d'anhydride carbonique conduit nécessairement à un mélange d'oxadiazines dont la séparation présente des difficultés considérables.

Le procédé découvert par la demanderesse ne présente pas ces inconvénients et permet la préparation facile d'une multitude d'oxadiazines 1,3,5 triones 2,4,6 moléculaires, portant des substituants identiques ou différents dans les positions 3 et 5 du noyau hétérocyclique, dans des conditions économiques.

Les composés suivant la présente invention ont des utilisations diverses et sont, entre autres, des intermédiaires dans la préparation de produits pharmaceutiques, d'herbicides sélectifs, de désinfectants; ils peuvent également être utilisés comme additifs

dans la fabrication de polymères et, en particulier, de polyuréthannes dans lesquels ils contribuent à la formation de liens allophanates et la production d'anhydride carbonique gazeux, utile dans la formation de mousses.

On illustre ces applications par les réactions suivantes conduisant à la formation d'allophanates, biurets et uréines à partir d'un sel de méthyl 3 oxadiazine 1,3,5 trione 2,4,6 et de chlorure d'allyle.

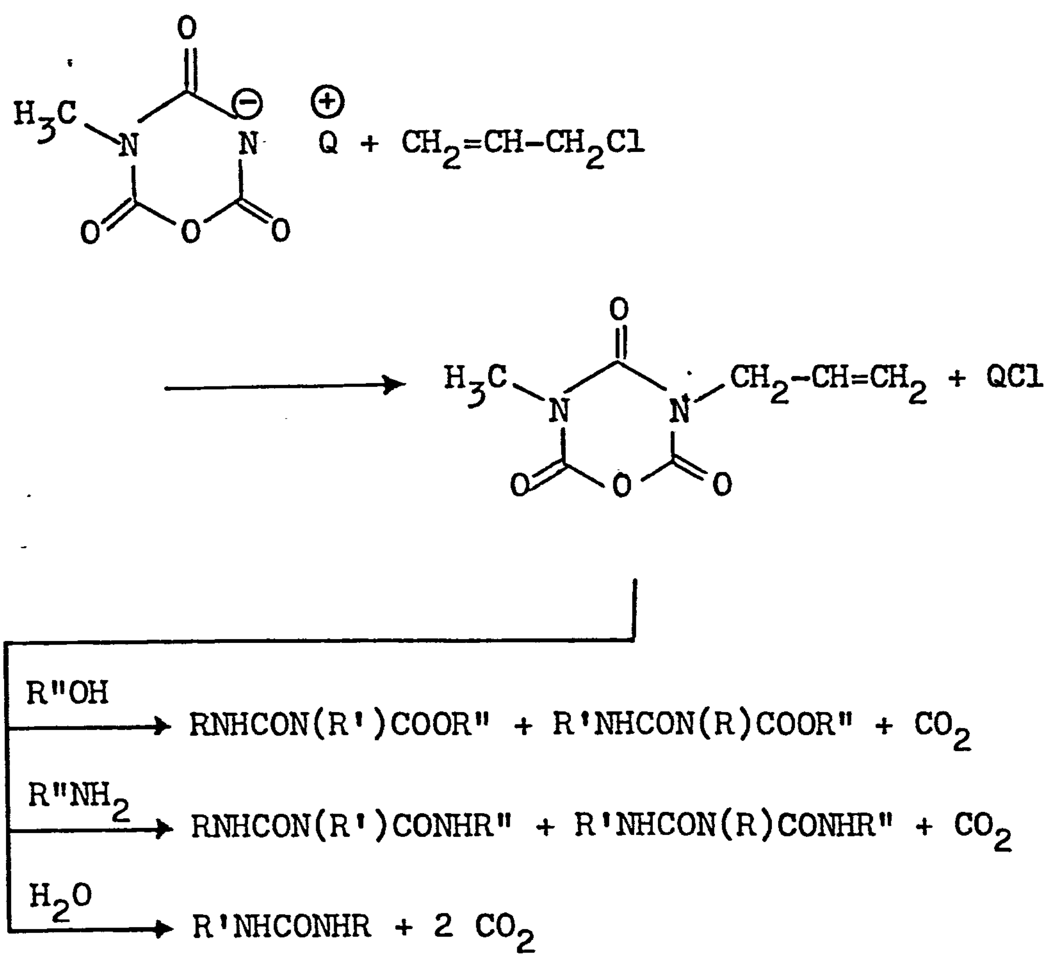


Dans ces réactions, R désigne un radical méthyle ($CH_3$), R' un radical allyle ($CH_2CH=CH_2$) et R" un radical phényle $C_6H_5$.

Parmi les biurets, allophanates et uréines que les composés de la présente invention permettent

de préparer, certains présentent des propriétés herbicides sélectives intéressantes.

La présente invention est basée sur la réaction d'isocyanates organiques avec un cyanate salin en présence d'anhydride carbonique. On a constaté, de manière inattendue, qu'en faisant réagir ces composés dans des conditions particulières l'on obtenait, non pas des isocyanurates ou des oxadiazines 1,3,5 triones moléculaires, mais des sels d'oxadiazines 1,3,5 triones 2,4,6 à partir desquels peuvent être préparées, de manière très simple, des oxadiazines 1,3,5 triones 2,4,6 moléculaires monosubstituées ou disubstituées symétriques ou asymétriques, ces derniers composés asymétriques répondant à la formule (II). Ces composés sont obtenus par le procédé suivant l'invention, avec de très bons rendements, à partir de réactifs peu coûteux et dans des conditions économiques.

Les sels d'oxadiazines 1,3,5 triones 2,4,6 de formule (I) suivant la présente invention sont obtenus, conformément à la présente invention, par réaction d'un isocyanate ou d'un polyisocyanate organique de formule

$$R\ (NCO)_p \qquad (III)$$

dans laquelle R et p ont les significations indiquées plus haut avec un cyanate salin de formule

$$Q\ (NCO)_n \qquad (IV)$$

dans laquelle Q désigne un métal ou un groupement contenant des fonctions ammonium, phosphonium ou arsonium non substituées ou mono-, bi-, tri- ou tétrasubstituées et n a la signification indiquée plus haut, et avec de l'anhydride carbonique.

La réaction A conduisant aux nouveaux composés de formule générale (I) est la suivante :

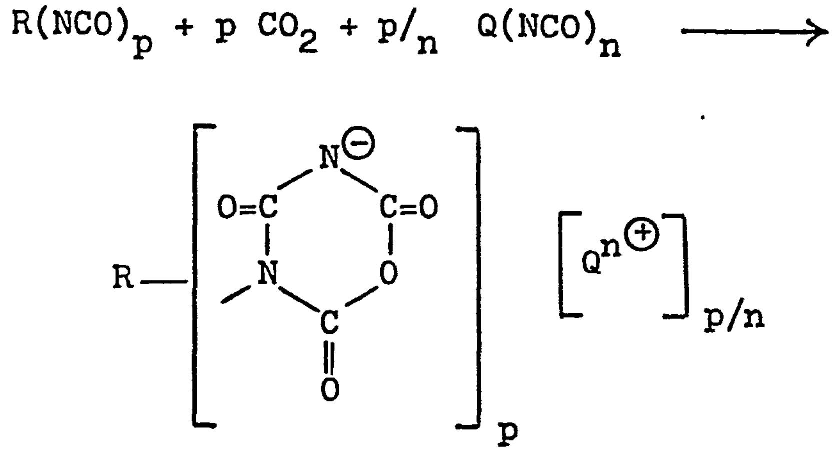


(A)

(I)

où R, Q, p et n ont les significations indiquées plus haut.

Dans le cas particulier de la préparation des sels d'oxadiazines 1,3,5 triones 2,4,6 de formule (I) dans laquelle n = 1 et p = 1, le procédé suivant l'invention implique la réaction B suivante :

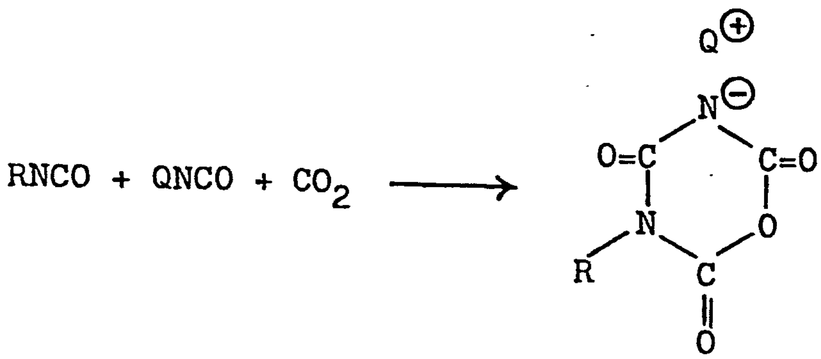


(B)

Le procédé suivant l'invention (A) peut s'effectuer éventuellement en présence d'un solvant organique n'interférant pas avec la réaction, à des températures comprises entre environ -80°C et +250°C et des pressions comprises entre environ 0,2 bar et 150 bars.

Dans le procédé (A) suivant l'invention, l'isocyanate organique de formule $R(NCO)_p$ peut être introduit tel quel dans le mélange de réaction ou être préparé par

toute méthode connue dans le solvant utilisé pour la préparation des sels d'oxadiazines 1,3,5 triones 2,4,6. Cette combinaison de la préparation de l'isocyanate et de sa transformation, dans le même solvant, en sels d'oxadiazines 1,3,5 triones 2,4,6 rend le procédé encore plus économique puisqu'il n'utilise pas d'isocyanate organique, réactif coûteux, comme matière première.

En particulier, la préparation de l'isocyanate organique peut se faire à partir d'un cyanate salin et d'un halogénure ou sulfate organique, éventuellement en présence d'un catalyseur, comme décrit par la réaction C suivante :

$$RX + QNCO \longrightarrow RNCO + QX \qquad (C)$$

où X désigne un halogène, un radical sulfate, alcoylsulfate ou arylsulfonyle.

La préparation de sels d'oxadiazines 1,3,5 triones 2,4,6 directement à partir d'un cyanate salin, d'un halogénure ou d'un sulfate organique et d'anhydride carbonique est représentée par la réaction D suivante :

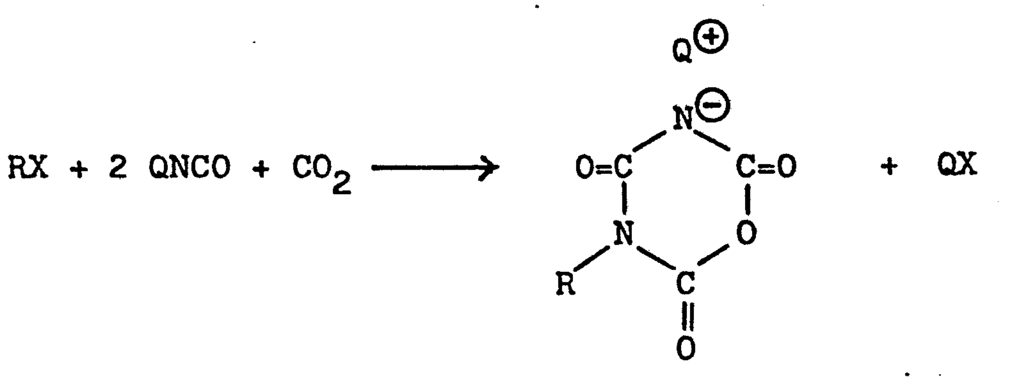

(D)

où R et X ont les significations indiquées plus haut.

Les matières premières utilisables, dans le cadre de la présente invention (réaction A), pour la préparation de sels d'oxadiazines 1,3,5 triones 2,4,6 par action d'un isocyanate organique avec un cyanate

salin et de l'anhydride carbonique sont les suivantes :

a. Les isocyanates organiques de formule générale $R(NCO)_p$ sont des mono- ou polyisocyanates aliphatiques primaires, secondaires ou tertiaires, cycloaliphatiques, arylaliphatiques et hétérocycliques éventuellement substitués; sont préférés les isocyanates organiques dans lesquels l'azote de la fonction isocyanate n'est pas lié directement à un carbone d'un noyau aromatique. Des exemples d'isocyanates organiques préférés dans la présente invention sont l'isocyanate de méthyle, l'isocyanate d'éthyle, le 1-isocyanatobutane, le 1-isocyanatodécane, l'isocyanate d'allyle, le 1-isocyanato octadéc-9-ène, l'hexaméthylène diisocyanate, le 1,4-diisocyanatobut-2-ène, l'isocyanate de vinyle, l'isocyanate d'isopropyle, le 2,5-diisocyanatohexane, l'isocyanato-isoheptadécane, l'isocyanate de t-butyle, le 5-méthyl-2,5,8-triisocyanatononane, l'isocyanate de cyclohexyle, le 1,3-diisocyanatocyclobutane, le 1,4-diisocyanatocyclohexane, le benzylisocyanate, le α,α'-diisocyanato-p-xylène, le 3-isocyanatofuranne ou leurs mélanges.

b. Les cyanates salins de formule générale $Q(NCO)_n$ sont des cyanates de métaux mono-, bi- ou trivalents ou des cyanates d'ammonium, de phosphonium ou d'arsonium non substitués ou mono-, bi-, tri- ou tétrasubstitués. Dans ces cyanates d'ammonium, de phosphonium ou d' arsonium, Q désigne

- soit un radical de formule :

$$(R^{I}\ R^{II}\ R^{III}\ Z)_n\ R^{IV} \qquad (V)$$

dans laquelle n a la signification donnée plus haut, Z désigne de l'azote, du phosphore ou de l'arsenic et $R^{I}$, $R^{II}$, $R^{III}$ et $R^{IV}$, qui sont identiques ou différents, désignent de l'hydrogène ou des radicaux

alcoyle, aryle, arylalcoyle ou hétérocycliques substitués ou non; $R^{I}$ et $R^{II}$, pris ensemble, peuvent aussi représenter un radical bivalent ;

- soit un radical de formule :

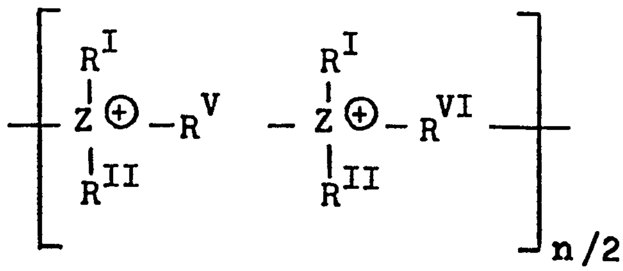

(VI)

dans laquelle Z, $R^{I}$ et $R^{II}$ ont les significations données ci-dessus, $R^{V}$ et $R^{VI}$ sont des radicaux bivalents et n a la signification donnée plus haut.

Le cyanate salin préféré dépend de la réactivité du système, de la température et du liquide dans lequel la préparation est réalisée.

Dans la plupart des cas, il est avantageux d'utiliser comme cyanate salin un cyanate de métal alcalin ou alcalino-terreux, de préférence les cyanates de sodium ou de potassium ou des cyanates d'ammonium, phosphonium ou arsonium tétrasubstitués ou leurs mélanges.

Les cyanates d'ammonium, phosphonium ou arsonium peuvent éventuellement être produits dans le solvant réactionnel, avant ou pendant la préparation, par toute méthode connue.

Des exemples de cyanates d'ammonium, phosphonium et arsonium tétrasubstitués utilisables dans le procédé suivant la présente invention sont le cyanate de tétraméthylammonium, le cyanate de tétraéthylammonium, le cyanate de tétrapropylammonium, le cyanate de tétrabutylammonium, le cyanate de tétraamylammonium, le cyanate de tétrahexylammonium, le cyanate de tributylméthylammonium, le cyanate de tricaprylméthylammonium,

le cyanate de dodécyltriméthylammonium, le cyanate de benzyltriméthylammonium, le cyanate de benzyltriéthylammonium, le cyanate de benzylisopropyldiméthylammonium, le cyanate de benzyltripropylammonium, le cyanate de benzyltributylammonium, le cyanate de phényltriméthylammonium, le cyanate de phényltriéthylammonium, le cyanate de méthyltributylphosphonium, le cyanate de tétrabutylphosphonium, le cyanate de tétraphénylphosphonium, le cyanate de benzyltriéthylarsonium, le cyanate de tétrapropylarsonium, le cyanate de tétraphénylarsonium, le cyanate de diméthylpipéridinium, le cyanate de méthylpyridinium ou leurs mélanges et leurs dérivés substitués.

Comme exemples d'autres cyanates utilisables dans le procédé suivant l'invention, on peut citer le cyanate de 1,4-bis(triméthylammonium)butane, le cyanate de 1,6-bis(triéthylammonium)hexane, le cyanate de 1,4-diméthyl-1,4-diaza-2,2,2-bicyclo-octane, les cyanates fixés sur une résine anionique du type AMBERLITE IRA 400 (FLUKA), l'ionène de formule :

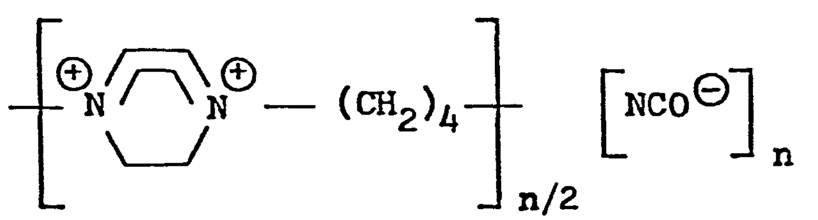

(VII)

et l'ionène de formule :

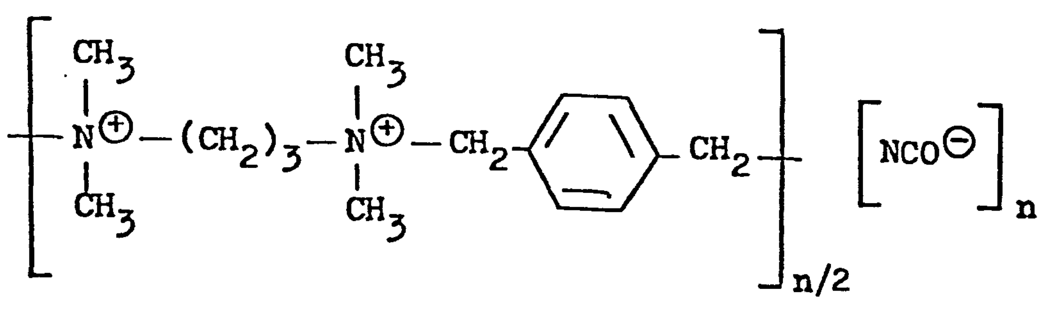

(VIII)

où n est compris entre 5 et 40,

ou leurs mélanges.

c. L'anhydrice carbonique utilisé dans la présente invention ne doit pas contenir comme impuretés des substances qui interfèrent avec la réaction, mais peut éventuellement être dilué par des substances inertes.

d. L'utilisation de catalyseurs n'est pas indispensable dans le procédé suivant la présente invention, qui permet des préparations avec de bons rendements dans des temps assez courts; des catalyseurs tels que $CaCl_2$, MgO peuvent cependant être utilisés dans des cas spécifiques.

Il est avantageux que les substances, telles que les cyanates, qui sont utilisées à l'état de matière solide soient sous la forme la plus divisée possible.

Les conditions de réaction, telles que la température, la pression et le solvant dans lequel se déroulent les réactions, ainsi que les rapports quantitatifs des substances à mettre en oeuvre, dépendent dans une certaine mesure l'une de l'autre et se choisissent, le cas échéant, selon le type de substance utilisée et la nature du produit désiré.

Le rapport quantitatif du cyanate salin à l'isocyanate organique et à l'anhydride carbonique peut être choisi dans de larges limites, ce rapport pouvant être aussi bien stoechiométrique que supérieur ou inférieur à la stoechiométrie. En général, on utilise, par équivalent de cyanate salin, 0,5 à 2 équivalents d'isocyanate organique, de préférence 0,8 à 1,2 équivalent d'isocyanate organique.

La quantité d'anhydride carbonique à utiliser est comprise entre 0,1 et 20 moles par équivalent de cyanate salin utilisé et de préférence entre 0,8 et 2 moles.

Le procédé suivant la présente invention, bien que réalisable en l'absence de solvant, est effectué de préférence dans un solvant organique inerte n'interférant pas avec la réaction. On peut utiliser aussi bien des solvants organiques apolaires ou peu polaires que des solvants organiques polaires. Comme solvants utilisables dans le procédé suivant la présente invention, on peut citer, par exemple, le tétrachlorure de carbone, le chloroforme, le dichlorométhane, le 1,2-dichloréthane, le 1,1-dichloréthane, le mélange d'isomères cis et trans du 1,2-dichloréthylène, l'o-dichlorobenzène, le dioxanne, l'acétate d'éthyle, le diméthoxyéthane, le tétrahydrofuranne, le toluène, l'α-méthylnaphtalène, l'acétone, la méthyléthylcétone, l'acétonitrile, le propionitrile, le benzonitrile, le cyanure de benzyle, le nitrobenzène, le nitrotoluène, le diméthylformamide, le diméthylacétamide, la tétraméthylurée, la N-méthylpyrrolidone, l'hexaméthylphosphotriamide, le diméthylsulfoxyde ainsi que leurs mélanges. Conviennent tout particulièrement les hydrocarbures chlorés, les éthers linéaires ou cycliques, les cétones et les nitriles.

La proportion de solvant dépend, dans une large mesure, du type de solvant et du rapport quantitatif des autres substances. En général, pour chaque partie en poids de réactifs, on peut utiliser jusque 50 parties en poids de solvant. Dans la plupart des cas, il est avantageux d'utiliser 0,2 à 25 parties en poids de solvant, de préférence de 1 à 10 parties en poids de solvant, pour une partie en poids de réactifs.

La préparation de sels d'oxadiazines 1,3,5 triones 2,4,6 suivant l'invention peut être réalisée à des températures comprises entre environ -80°C et +250°C. Dans la plupart des cas, on peut utiliser des températures comprises entre -20°C et 150°C et, plus spécialement, des températures comprises entre 0°C et 70°C.

La pression d'anhydride carbonique dans l'enceinte réactionnelle n'est pas un facteur limitatif, pourvu que le liquide contienne de l'anhydride carbonique dissous. Pour atteindre cet objectif, il convient que la pression partielle de l'anhydride carbonique dans l'enceinte réactionnelle soit comprise entre 0,2 bar et 150 bars et de préférence entre 1 et 60 bars. L'enceinte réactionnelle peut éventuellement contenir en plus un gaz inerte, tel que l'azote et l'argon.

La mise en oeuvre des réactifs et du solvant peut s'effectuer de diverses manières. En particulier, les réactifs et le solvant peuvent être introduits dans le réacteur conjointement, successivement et/ou progressivement. Ainsi, la quantité totale d'anhydride carbonique utile peut être introduite dans le réacteur dans sa totalité dès l'origine ou être alimentée en cours de réaction.

Le temps de réaction varie dans une large mesure suivant la réactivité des réactifs, la température, la nature du solvant et la présence éventuelle de catalyseurs. En général, le temps de réaction est compris entre 0,5 sec. et 24 heures et, le plus souvent, entre 1 sec. et 10 heures.

Les rendements en sels d'oxadiazines 1,3,5 triones 2,4,6 préparés suivant la présente invention sont en général élevés et peuvent être supérieurs à 90 % après 1 minute de réaction à 25°C et sous une pression de 1 bar.

La transformation des sels d'oxadiazines 1,3,5 triones 2,4,6 en oxadiazines 1,3,5 triones 2,4,6 moléculaires symétriques ou asymétriques (formule II) s'effectue, conformément à l'invention, par réaction d'un composé de formule (I) avec un composé de formule

R'Y (IX)

dans laquelle R' désigne un radical monovalent ou polyvalent, aliphatique, cycloaliphatique, arylaliphatique ou hétérocyclique éventuellement substitué, ou de l'hydrogène, un halogène, un radical chlorocarbonyle,

carbamoyle, sulfamoyle, sulfonyle et Y désigne un halogène, un radical sulfate, un reste d'un acide organique ou inorganique, un radical alcoxy ou un radical amide.

Ainsi, conformément à l'invention, à partir de sels d'oxadiazines 1,3,5 triones 2,4,6 de formule générale (I), on obtient des oxadiazines 1,3,5 triones 2,4,6 moléculaires de formule générale (II) par la réaction E suivante :

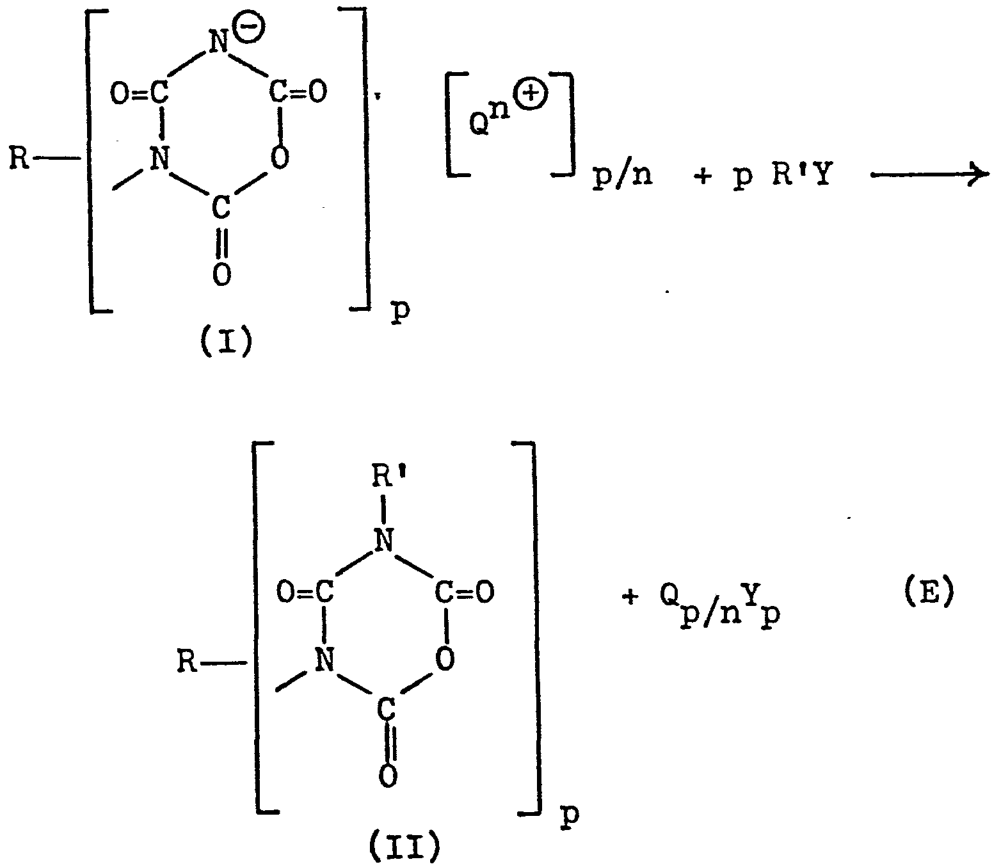


(I)

(II)

(E)

où

R désigne un radical monovalent ou polyvalent, aliphatique, cycloaliphatique, arylaliphatique ou hétérocyclique éventuellement substitué ;

Q désigne un métal ou un groupement contenant des fonctions ammonium, phosphonium ou arsonium non substituées ou mono-, bi-, tri- ou tétrasubstituées ;

p est un nombre entier égal ou supérieur à 1 ;

n désigne le nombre d'oxydation du métal ou le nombre de fonctions ammonium, phosphonium ou arsonium présentes dans le groupement Q ;

R' désigne un radical monovalent ou polyvalent, aliphatique, cycloaliphatique, arylaliphatique ou hétérocyclique éventuellement substitué, ou de l'hydrogène, un halogène, un radical chlorocarbonyle, carbamoyle, sulfamoyle, sulfonyle et

Y désigne un halogène, un radical sulfate, un reste d'un acide organique ou inorganique, un radical alcoxy ou un radical amide.

Cette réaction E permet la préparation d'oxadiazines 1,3,5 triones 2,4,6 moléculaires monosubstituées ou disubstituées symétriques ou asymétriques. Les composés monosubstitués ou disubstitués asymétriques sont nouveaux et sont compris dans la présente invention.

En utilisant, comme réactif de formule générale R'Y, un acide, on obtient des oxadiazines monosubstituées répondant à la formule (X) ci-dessous :

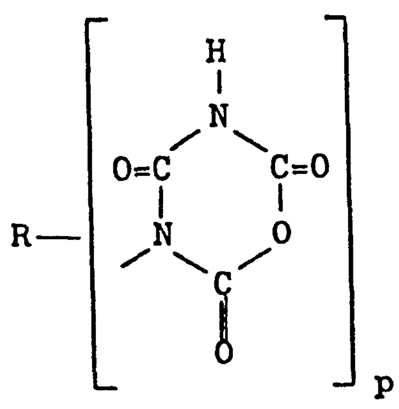

(X)

Comme acide, on peut utiliser tout acide organique ou minéral susceptible de céder un proton aux sels d'oxadiazines 1,3,5 triones 2,4,6 qui possèdent des propriétés basiques, par exemple un acide choisi parmi l'acide chlorhydrique, l'acide sulfurique, l'acide acétique, l'acide benzoïque, l'acide formique et l'acide oxalique. La neutralisation peut être réalisée indifféremment dans le solvant organique de la préparation, dans l'eau, dans un autre solvant ou dans un mélange de solvants.

Pour la préparation d'oxadiazines 1,3,5 triones 2,4,6 disubstituées symétriques ou asymétriques (répondant à la formule générale II), on utilise avantageusement, comme réactifs de formule générale R'Y, des halogénures ou des sulfates organiques renfermant de 1 à 30 atomes de carbone, de préférence de 1 à 18 atomes de carbone, pouvant être substitués par des groupements n'interférant pas avec la réaction.

Comme exemples d'halogénures et de sulfates organiques utilisables dans la présente invention, on peut citer le sulfate de diméthyle, le sulfate de diéthyle, le chlorure de benzyle, le chlorure d'allyle, le 1-chloro-but-2-ène, le chlorure de cyclohexyle, le chlorure de méthyle, l'iodure de méthyle, l'α-chloro-p-nitrotoluène, le 1-chlorohexane, l'α-chloro-p-trifluorométhyltoluène, le chlorure d'acétyle, le chlorure de benzoyle, le chlorure de p-toluènesulfonyle, le chlorure d'oxalyle, les chlorures de phtalyle, le 1,4-dichlorobutène, l'α,α'-dichloro-m-xylène, l'α,α'-dichloro-p-xylène, le 1,6-dichlorohexane ou leurs combinaisons et leurs dérivés substitués.

Suivant une variante du procédé de l'invention (réaction E), les oxadiazines 1,3,5 triones 2,4,6 moléculaires disubstituées symétriques ou asymétriques peuvent être obtenues directement par réaction d'un isooyanate organique, d'un cyanate salin, d'anhydride carbonique et du réactif de formule générale R'Y.

Cette variante est représentée par la réaction F suivante :

$$R(NCO)_p + p/_n\ Q(NCO)_n + {}_pCO_2 + {}_pR'Y \longrightarrow$$

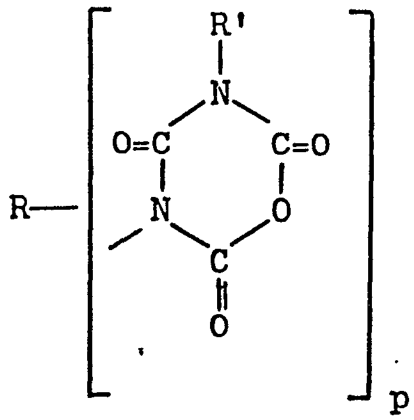

$+\ p/_n\ QY_n$ (F)

L'isocyanate de formule $R(NCO)_p$ intervenant dans cette préparation peut être introduit tel quel dans le mélange de réaction.

On peut aussi, suivant une autre particularité de l'invention, former l'isocyanate de formule $R(NCO)_p$ dans le mélange même de réaction, à partir du cyanate QNCO et d'un halogénure ou d'un sulfate organique de formule $RX_p$.

En faisant réagir ensemble le cyanate QNCO, les halogénures ou sulfates organiques RX et R'Y et l'anhydride carbonique, l'on peut associer de cette manière, dans un seul mélange de réaction, les réactions de formation de l'isocyanate, de la transformation de l'isocyanate en sels d'oxadiazines 1,3,5 triones 2,4,6 et de la transformation de ces derniers en oxadiazines 1,3,5 triones 2,4,6 moléculaires disubstituées symétriques ou asymétriques. Cette préparation est représentée par la réaction globale suivante (G) :

RX + 2 QNCO + $CO_2$ + R'Y ⟶

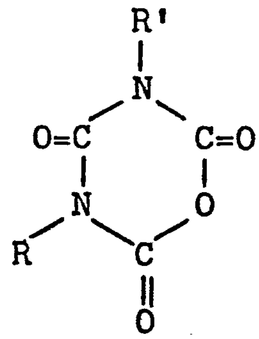 + QY + QX (G)

L'invention est décrite plus en détail à l'aide des exemples qui suivent, destinés à illustrer l'invention sans la limiter.

Les exemples décrits ont été réalisés en discontinu dans des réacteurs du type Grignard; il est évident que des réacteurs opérant en continu peuvent aussi être utilisés.

Les produits obtenus ont été identifiés par détermination de leurs compositions élémentaires, titrage potentiométrique, analyse par spectroscopie I.R., de résonnance magnétique nucléaire du $^{13}C$ et spectroscopie de masse.

EXEMPLE 1

L'appareil est un réacteur en verre de 0,5 l muni de 5 ouvertures, équipé d'un agitateur magnétique, d'un condenseur à reflux, d'un thermomètre, d'un dispositif permettant l'introduction d'un liquide et d'une arrivée de gaz par un diffuseur plongeant dans la solution; la température est réglée à l'aide d'un thermostat.

On introduit dans le réacteur 22,4 g de cyanate de tétrapropylammonium (0,10 mole) et 250 ml de dichlorométhane anhydre. Cette solution est maintenue saturée en anhydride carbonique; après dissolution des réactifs, on ajoute au mélange, sous agitation et à 25°C, 5,7 g de

méthyl isocyanate (0,1 mole); après 2 minutes de réaction, le solvant est distillé sous vide. Après lavage au tétrachlorure de carbone, on obtient 27,6 g de méthyl-3-oxadiazinate 1,3,5 trione 2,4,6 de tétrapropylammonium (rendement 85 ‰) tombant dans la formule (I).

EXEMPLE 2

On opère comme dans l'exemple 1, sans distillation du solvant. A la solution de sel de méthyl-3-oxadiazine 1,3,5 trione 2,4,6, on additionne 15,1 g de sulfate de méthyle (0,12 mole). Après 10 minutes de réaction, à 25°C, le solvant est évaporé. Après lavage à l'eau et au tétrachlorure de carbone, on obtient 14,6 g de diméthyl-3,5-oxadiazine 1,3,5 trione 2,4,6 (rendement 92 ‰) qui est une oxadiazine 1,3,5 trione disubstituée symétrique.

EXEMPLE 3

On opère comme dans l'exemple 1. A la solution obtenue de sel de méthyl-3-oxadiazine 1,3,5 trione 2,4,6, on additionne 15,2 g de chlorure de benzyle (0,12 mole). Après 3 heures de réaction, à 60°C, on isole 19,4 g de méthyl-3-benzyl-5-oxadiazine 1,3,5 trione 2,4,6 (rendement 83 ‰), qui est une oxadiazine 1,3,5 trione disubstituée asymétrique tombant dans la formule (III).

EXEMPLE 4

On opère avec le réacteur décrit dans l'exemple 1. On introduit 34,1 g de cyanate de tétraamylammonium (0,1 mole) et 250 ml de tétrahydrofuranne anhydre; cette solution est maintenue saturée en anhydride carbonique; on ajoute progressivement, en 5 minutes, 7,1 g d'isocyanate d'éthyle (0,1 mole), sous agitation et à 25°C. Après 10 minutes de réaction à 25°C, le solvant est évaporé; après lavage à l'éther, on obtient 41 g de éthyl-3 oxadiazinate 1,3,5 trione 2,4,6 de tétraamylammonium (rendement 90 ‰) tombant dans la formule (I).

EXEMPLE 5

On opère comme dans l'exemple 4, puis la solution de sel d'oxadiazine est additionnée de 9,4 g de chlorure d'acétyle (0,12 mole). Après distillation sous vide, le solide est lavé au tétrachlorure de carbone; on obtient 17 g de méthyl-3-acétyl-5-oxadiazine 1,3,5 trione 2,4,6 (rendement 85 %) tombant dans la formule (II).

EXEMPLE 6

Dans un réacteur en acier inoxydable de 0,5 l, muni d'un agitateur et d'un dispositif pour l'introduction de gaz et de liquide, on introduit 24,3 g de cyanate de potassium (0,3 mole) finement divisé, 5,7 g de $CH_3NCO$ (0,1 mole) et 250 ml d'acétonitrile anhydre; la pression de $CO_2$ dans le réacteur est maintenue constante à 15 bars. La réaction est poursuivie pendant 6 heures, sous agitation, à 70°C. La solution est filtrée à chaud; le filtrat est distillé sous vide. L'extraction à l'eau de ce solide permet la séparation du méthyl-3-oxadiazinate 1,3,5 trione 2,4,6 de potassium, soluble dans l'eau, du triméthylisocyanurate insoluble dans l'eau. On isole ainsi 6,4 g de méthyl-3-oxadiazinate de potassium (rendement 35 %) tombant dans la formule (I).

Dans une autre expérience réalisée dans les mêmes conditions, la solution aqueuse contenant le méthyl-3-oxadiazinate de potassium a été neutralisée par l'acide chlorhydrique. On isole ensuite 4,3 g de méthyl-3-oxadiazine 1,3,5 trione 2,4,6 (rendement 30 %) tombant dans la formule (II).

EXEMPLE 7

Dans le réacteur décrit dans l'exemple 1, on introduit 34,6 g de cyanate de tétraamylammonium (0,1 mole) et 250 ml de tétrahydrofuranne; la solution est maintenue saturée en $CO_2$ à la pression atmosphérique. On introduit 8,4 g d'hexaméthylènediisocyanate (0,05 mole) progressivement dans un laps de temps de 5 minutes; après 10 minutes de réaction, le solvant est évaporé et le solide obtenu lavé au tétrachlorure de carbone. On obtient ainsi 44 g (rendement 94 %) du bis-oxadiazinate de tétraamylammonium de formule

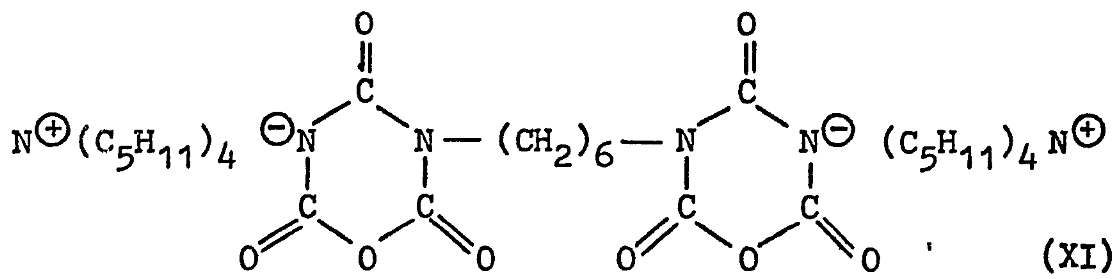 , (XI)

Ce composé tombe dans la formule (I).

Dans une autre expérience, conduite de la même manière, la solution de bis-oxadiazinate a été mise en présence de 0,12 mole de diméthylsulfate; après 10 minutes de réaction à 25°C, la solution est distillée sous vide; le résidu solide est lavé à l'eau et au tétrachlorure de carbone; on obtient ainsi 16,6 g (rendement 90 %) de la bis-oxadiazine de formule

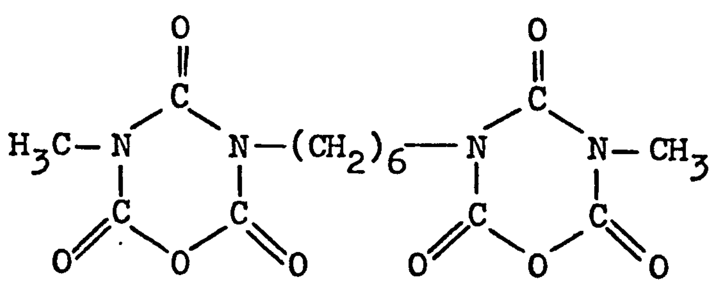 (XII)

EXEMPLE 8

Cet exemple illustre la possibilité de coupler la production des isocyanates organiques avec la production des oxadiazines 1,3,5 triones 2,4,6.

Dans le réacteur décrit dans l'exemple 1, on introduit 56,9 g de cyanate de tétrabutylammonium (0,2 mole) et 200 ml d'acétonitrile; la solution est maintenue

saturée en $CO_2$ sous une pression de 1 bar à 25°C. On y introduit progressivement et sous agitation en un laps de temps de 10 minutes, 0,09 mole de sulfate de méthyle dissous dans 50 ml d'acétonitrile à débit constant. Après 10 minutes de réaction supplémentaire, on vérifie la disparition de la totalité du sulfate de méthyle engagé et on détermine la quantité de cyanate salin restant; dans l'expérience illustrant cet exemple, il subsiste à ce moment 0,02 mole de cyanate salin dans la solution. La solution est traitée comme dans l'exemple 1. On isole ainsi 21,2 g de méthyl-3-oxadiazinate 1,3,5 trione 2,4,6 de tétrabutylammonium, tombant dans la formule (I).

EXEMPLE 9

On opère avec les mêmes réactifs et dans les mêmes conditions que dans l'exemple 8. Puis dans ce cas, à la solution obtenue dans l'exemple 8, on ajoute 0,12 mole de bromure de benzyle. Le mélange réactionnel est maintenu sous agitation pendant 2 heures à 50°C. On obtient 11,5 g de méthyl-3 benzyl-5 oxadiazine 1,3,5 trione 2,4,6 tombant dans la formule (II).

EXEMPLE 10

On opère avec l'appareil décrit dans l'exemple 1. On introduit dans le réacteur 6 g de cyanate de potassium finement divisé et 250 ml de diméthylsulfoxyde. Cette suspension est saturée en anhydride carbonique. On introduit, sous agitation et à 25°C, 0,08 mole d'isocyanate de méthyle; le mélange réactionnel est maintenu saturé en $CO_2$. Après 5 minutes de réaction à 25°C, on obtient 11 g (rendement 85 %) de méthyl-3-oxadiazinate 1,3,5 trione 2,4,6 de potassium tombant dans la formule (I).

EXEMPLE 11

On opère avec le réacteur décrit dans l'exemple 1. On introduit 0,1 mole de cyanate de tétraphénylarsonium et 250 ml de dichlorométhane; après saturation en anhydride

carbonique, on ajoute progressivement 0,1 mole d'isocyanate de benzyle. Après 10 minutes de réaction, sous agitation à 50°C, on obtient le 3-benzyloxadiazinate 1,3,5 trione 2,4,6 de tétraphénylarsonium tombant dans la formule (I), avec un rendement de 37 %.

EXEMPLE 12

On opère avec le réacteur décrit dans l'exemple 1. On introduit 30 g de cyanate de tributylbenzylammonium (0,094 mole) et 100 ml de tétrahydrofuranne anhydre; cette solution est maintenue saturée en anhydride carbonique; on ajoute progressivement, en 1 minute, 8,3 ml d'éthylisocyanate (0,105 mole) sous agitation et à 25°C. Après 5 minutes de réaction à 25°C, le cyanate de tributylbenzylammonium a totalement disparu. On obtient 36 g d'éthyl-3-oxadiazinate 1,3,5 trione 2,4,6 de tributylbenzylammonium tombant dans la formule (I).

EXEMPLE 13

On opère comme dans l'exemple 12, puis la solution de sel d'oxadiazine est additionnée de 11,5 ml de bromoacétate d'éthyle (0,104 mole). Après distillation sous vide, le solide est lavé à l'eau et au tétrachlorure de carbone; on obtient 17,3 g d'éthyl-3-éthylacéto-5-oxadiazine 1,3,5 trione 2,4,6 tombant dans la formule (II).

EXEMPLE 14

On opère avec un réacteur similaire à celui décrit dans l'exemple 1, mais dont le volume est de 1 litre. On introduit dans le réacteur 165 g de cyanate de tributylbenzylammonium (0,518 mole) et 500 ml de tétrahydrofuranne anhydre. Cette solution est maintenue saturée en anhydride carbonique. On ajoute au mélange, sous agitation et à 25°C, 65 ml de butylisocyanate (0,577 mole); après 5 minutes de réaction, le solvant est distillé sous vide. Après un lavage au tétrachlorure de carbone, on obtient 215 g de butyl-3-oxadiazinate 1,3,5 trione 2,4,6 de tributylbenzylammonium (rendement 90 %) tombant dans la formule (I).

EXEMPLE 15

On opère comme dans l'exemple 14, sans distillation du solvant. A la solution de sel de butyl-3-oxadiazine 1,3,5 trione 2,4,6 de tributylbenzylammonium, on additionne 72 g de sulfate de méthyle (0,57 mole). Après 5 heures de réaction à 25°C, le solvant est évaporé. Après lavage à l'eau et au tétrachlorure de carbone, on obtient 88 g de butyl-3 méthyl-5 oxadiazine 1,3,5 trione 2,4,6 (rendement 81 %) tombant dans la formule (II).

0098005

## R E V E N D I C A T I O N S

1. A titre de nouveaux composés, les sels d'oxadiazines 1,3,5 triones 2,4,6 répondant à la formule générale suivante :

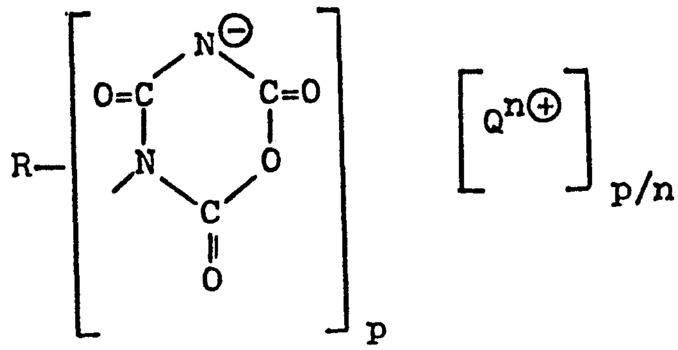

(I)

dans laquelle

R désigne un radical hydrocarboné, substitué ou non mono- ou polyvalent ;

Q désigne un métal ou un groupement contenant un nombre n de fonctions ammonium, phosphonium ou arsonium, non substituées ou mono-, di-, tri- ou tétrasubstituées ;

p est un nombre entier au moins égal à 1 ;

n désigne le nombre d'oxydation du métal ou le nombre de fonctions ammonium, phosphonium ou arsonium présentes dans le groupement Q.

2. A titre de nouveaux composés, les oxadiazines 1,3,5 triones 2,4,6 moléculaires asymétriques répondant à la formule générale suivante :

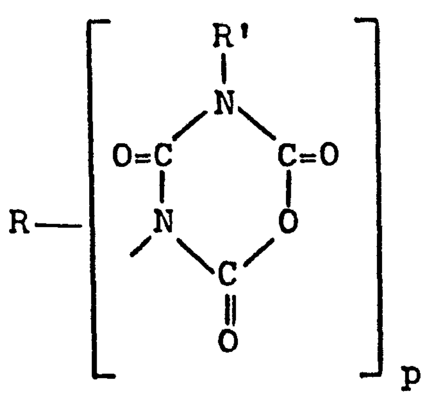

(II)

dans laquelle R et p ont la même signification que dans les composés de formule générale (I) et R', qui est différent de R, désigne un radical hydrocarboné éventuellement substitué, l'hydrogène, un halogène, un radical chlorocarbonyle, carbamoyle, sulfamoyle ou sulfonyle.

3. Procédé de préparation de sels d'oxadiazines 1,3,5 triones 2,4,6 répondant à la formule générale (I) suivant la revendication 1, caractérisé en ce qu'on fait réagir un isocyanate ou polyisocyanate organique de formule

$$R\ (NCO)_p \qquad (III)$$

dans laquelle R et p ont les significations indiquées plus haut, avec un cyanate salin de formule

$$Q\ (NCO)_n \qquad (IV)$$

dans laquelle Q et n ont les significations indiquées ci-avant et avec de l'anhydride carbonique.

4. Procédé suivant la revendication 3, caractérisé en ce qu'on prépare l'isocyanate organique de formule

$$R\ (NCO)_p \qquad (III)$$

dans laquelle R et p ont les significations indiquées plus haut, in situ, par réaction d'un cyanate salin de formule

$$Q\ (NCO)_n \qquad (IV)$$

dans laquelle Q a les significations indiquées plus haut et n est au moins égal à 1, avec un halogénure ou sulfate organique de formule

$$RX_p \qquad (XIII)$$

dans laquelle R et p ont les significations indiquées plus haut et X désigne un halogène ou un radical sulfate,

alcoylsulfate ou arylsulfonyle, éventuellement en présence d'un catalyseur.

5. Procédé suivant la revendication 4, caractérisé en ce qu'on prépare les composés de formule (I) par réaction d'un halogénure ou sulfate organique de formule

$$RX_p \qquad (XIII)$$

dans laquelle R, p et X ont les significations indiquées dans les revendications 1 et 4 avec un cyanate salin de formule

$$Q\,(NCO)_n \qquad (IV)$$

dans laquelle Q et n ont les significations indiquées dans la revendication 4 et avec de l'anhydride carbonique.

6. Procédé suivant l'une quelconque des revendications 3 à 5, caractérisé en ce qu'on utilise comme isocyanate organique de formule

$$R\,(NCO)_p \qquad (III)$$

dans laquelle R et p ont les significations indiquées plus haut, au moins un isocyanate choisi parmi les mono- ou polyisocyanates aliphatiques primaires, secondaires ou tertiaires, cycloaliphatiques, arylaliphatiques et hétérocycliques éventuellement substitués.

7. Procédé suivant la revendication 6, caractérisé en ce qu'on utilise comme isocyanate organique de formule $R(NCO)_p$ un composé choisi parmi l'isocyanate de méthyle, l'isocyanate d'éthyle, le 1-isocyanatobutane, le 1-isocyanatodécane, l'isocyanate d'allyle, le 1-isocyanato octadéc-9-ène, l'hexaméthylène diisocyanate, le 1,4-diisocyanatobut-2-ène, l'isocyanate de vinyle, l'isocyanate d'isopropyle, le 2,5-diisocyanatohexane, l'isocyanato-isoheptadécane, l'isocyanate de t-butyle, le 5-méthyl-2,5,8-triisocyanatononane, l'isocyanate de

cyclohexyle, le 1,3-diisocyanatocyclobutane, le 1,4-diisocyanatocyclohexane, le benzylisocyanate, l'α,α'-diisocyanato-p-xylène, le 3-isocyanatofuranne ou leurs mélanges.

8. Procédé suivant l'une quelconque des revendications 3 à 7, caractérisé en ce qu'on utilise un cyanate salin de formule

$$Q\ (NCO)_n \qquad (IV)$$

dans laquelle n est au moins égal à 1 et Q désigne un métal alcalin ou alcalino-terreux.

9. Procédé suivant la revendication 8, caractérisé en ce que le métal est du sodium ou du potassium.

10. Procédé suivant l'une quelconque des revendications 3 à 7, caractérisé en ce qu'on utilise un cyanate salin de formule

$$Q\ (NCO)_n \qquad (IV)$$

dans laquelle n est au moins égal à 1 et Q désigne un groupement contenant un nombre n de fonctions ammonium, phosphonium ou arsonium

- soit de formule

$$(R^{I}\ R^{II}\ R^{III}\ Z)_n\ R^{IV} \qquad (V)$$

dans laquelle n a la signification donnée plus haut, Z désigne de l'azote, du phosphore ou de l'arsenic et $R^{I}$, $R^{II}$, $R^{III}$ et $R^{IV}$, qui sont identiques ou différents, désignent de l'hydrogène ou des radicaux alcoyle, aryle, arylalcoyle ou hétérocycliques substitués ou non; $R^{I}$ et $R^{II}$, pris ensemble, peuvent aussi représenter un radical bivalent ;

- soit de formule

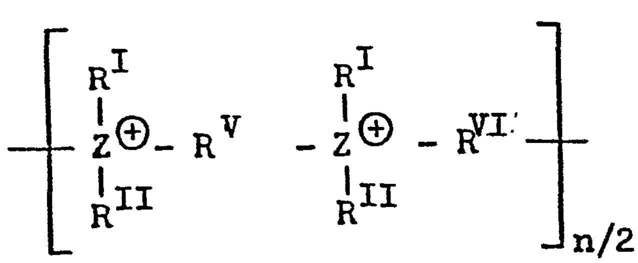

(VI)

dans laquelle Z, $R^{I}$ et $R^{II}$ ont les significations données ci-dessus, $R^{V}$ et $R^{VI}$ sont des radicaux bivalents et n a la signification donnée plus haut.

11. Procédé suivant la revendication 10, caractérisé en ce qu'on utilise un cyanate salin choisi parmi le cyanate de tétraméthylammonium, le cyanate de tétraéthylammonium, le cyanate de tétrapropylammonium, le cyanate de tétrabutylammonium, le cyanate de tétraamylammonium, le cyanate de tétrahexylammonium, le cyanate de tributylméthylammonium, le cyanate de tricaprylméthylammonium, le cyanate de dodécyltriméthylammonium, le cyanate de benzyltriméthylammonium, le cyanate de benzyltriéthylammonium, le cyanate de benzyltributylammonium, le cyanate de benzylisopropyldiméthylammonium, le cyanate de benzyltripropylammonium, le cyanate de phényltriméthylammonium, le cyanate de phényltriéthylammonium, le cyanate de méthyltributylphosphonium, le cyanate de tétrabutylphosphonium, le cyanate de tétraphénylphosphonium, le cyanate de benzyltriéthylarsonium, le cyanate de tétrapropylarsonium, le cyanate de tétraphénylarsonium, le cyanate de diméthylpipéridinium, le cyanate de méthylpyridinium ou leurs mélanges et leurs dérivés substitués.

12. Procédé suivant la revendication 10, caractérisé en ce qu'on utilise un cyanate salin choisi parmi le cyanate de 1,4-bis(triméthylammonium)butane, le cyanate de 1,6-bis(triéthylammonium)hexane, le cyanate de 1,4-diméthyl-1,4-diaza-2,2,2-bicyclo-octane, les cyanates fixés sur une résine anionique du type AMBERLITE IRA 400 (FLUKA),
l'ionène de formule :

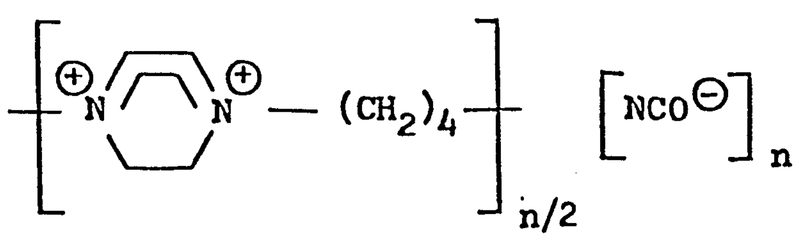

(VII)

et l'ionène de formule :

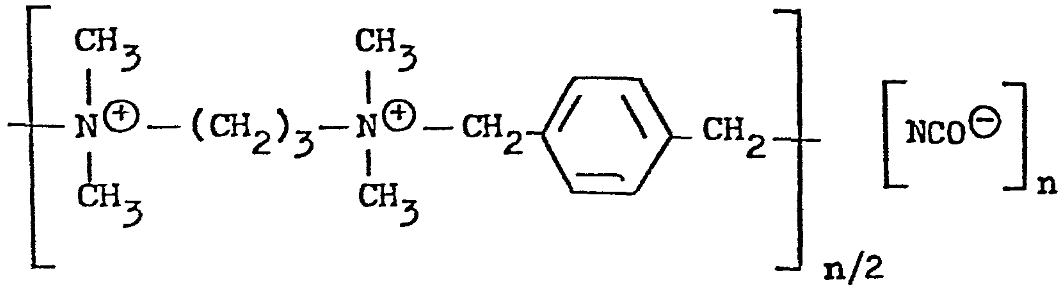

(VIII)

où n est compris entre 5 et 40, ou leurs mélanges.

13. Procédé suivant l'une quelconque des revendications 3 à 12, caractérisé en ce qu'on opère en présence d'un solvant organique n'interférant pas avec la réaction, à des températures comprises entre environ -80°C et +250°C et des pressions comprises entre environ 0,2 bar et 150 bars.

14. Procédé suivant l'une quelconque des revendications 3 à 13, caractérisé en ce qu'on utilise, par équivalent de cyanate salin, 0,5 à 2 équivalents d'isocyanate organique.

15. Procédé suivant la revendication 14, caractérisé en ce qu'on utilise, par équivalent de cyanate salin, 0,8 à 1,2 équivalent d'isocyanate organique.

16. Procédé suivant l'une quelconque des revendications 3 à 15, caractérisé en ce qu'on utilise une quantité d'anhydride carbonique comprise entre 0,1 et 20 moles par équivalent de cyanate salin.

17. Procédé suivant la revendication 16, caractérisé en ce qu'on utilise une quantité d'anhydride carbonique comprise entre 0,8 et 2 moles par équivalent de cyanate salin.

18. Procédé suivant la revendication 13, caractérisé en ce qu'on utilise un solvant choisi parmi

le tétrachlorure de carbone, le chloroforme, le dichlorométhane, le 1,2-dichloréthane, le 1,1-dichloréthane, le mélange d'isomères cis et trans du 1,2-dichloréthylène, l'o-dichlorobenzène, le dioxanne, l'acétate d'éthyle, le diméthoxyéthane, le tétrahydrofuranne, le toluène, l'α-méthylnaphtalène, l'acétone, la méthyléthylcétone, l'acétonitrile, le propionitrile, le benzonitrile, le cyanure de benzyle, le nitrobenzène, le nitrotoluène, le diméthylformamide, le diméthylacétamide, la tétraméthylurée, la N-méthylpyrrolidone, l'hexaméthylphosphotriamide, le diméthylsulfoxyde ainsi que leurs mélanges; le solvant étant choisi, de préférence, parmi les hydrocarbures chlorés, les éthers linéaires ou cycliques, les cétones et les nitriles.

19. Procédé de préparation d'oxadiazines 1,3,5 triones 2,4,6 moléculaires, caractérisé en ce qu'on fait réagir un sel d'oxadiazine 1,3,5 trione 2,4,6 suivant la revendication 1 avec un réactif de formule

$$R'Y \qquad (IX)$$

dans laquelle R' désigne un radical monovalent ou polyvalent, aliphatique, cycloaliphatique, arylaliphatique ou hétérocyclique éventuellement substitué, ou de l'hydrogène, un halogène, un radical chlorocarbonyle, carbamoyle, sulfamoyle, sulfonyle et Y désigne un halogène, un radical sulfate, un reste d'un acide organique ou inorganique, un radical alcoxy ou un radical amide.

20. Procédé suivant la revendication 19, caractérisé en ce qu'on utilise un réactif de formule R'Y constitué par un acide organique ou minéral susceptible de céder un proton aux sels d'oxadiazines 1,3,5 triones 2,4,6 de formule (I) qui possèdent des propriétés basiques.

21. Procédé suivant la revendication 20, caractérisé en ce qu'on utilise un acide choisi parmi l'acide chlorhydrique, l'acide sulfurique, l'acide acétique, l'acide benzoïque, l'acide formique et l'acide oxalique.

22. Procédé suivant la revendication 19, caractérisé en ce qu'on utilise un réactif de formule R'Y constitué par un halogénure ou un sulfate organique contenant 1 à 30 atomes de carbone.

23. Procédé suivant la revendication 22, caractérisé en ce que l'halogénure ou sulfate organique contient de 1 à 18 atomes de carbone.

24. Procédé suivant la revendication 23, caractérisé en ce qu'on utilise un halogénure ou sulfate organique choisi parmi le sulfate de diméthyle, le sulfate de diéthyle, le chlorure de benzyle, le chlorure d'allyle, le 1-chloro-but-2-ène, le chlorure de cyclohexyle, le chlorure de méthyle, l'iodure de méthyle, l'α-chloro-p-nitrotoluène, le 1-chlorohexane, l'α-chloro-p-trifluoro-méthyltoluène, le chlorure d'acétyle, le chlorure de benzoyle, le chlorure de p-toluènesulfonyle, le chlorure d'oxalyle, les chlorures de phtalyle, le 1,4-dichlorobutène, l'α,α'-dichloro-m-xylène, l'α,α'-dichloro-p-xylène, le 1,6-dichlorohexane ou leurs combinaisons et leurs dérivés substitués.

25. Procédé suivant l'une quelconque des revendications 19 à 24, caractérisé en ce qu'on prépare une oxadiazine 1,3,5 trione 2,4,6 moléculaire par réaction d'un isocyanate organique de formule

$$R\ (NCO)_p \qquad (III)$$

dans laquelle R et p ont les significations indiquées plus haut, avec un cyanate salin de formule

$$Q\ (NCO)_n \qquad (IV)$$

dans laquelle Q et n ont les significations indiquées plus haut, avec de l'anhydride carbonique et avec un réactif de formule

$$R'Y \qquad (IX)$$

dans laquelle R' et Y ont les significations indiquées dans l'une quelconque des revendications 22 à 24.